# EUROPEAN PATENT APPLICATION

(11) **EP 1 459 685 A1**
(43) Date of publication of application: **22.09.2004**
(21) Application number: 04425182.5
(22) Date of filing: 17.03.2004
(51) Int. Cl.: A61B 6/14, A61B 6/00

(54) **Improved radiogenic apparatus, particularly for orthopantomography of dental arches, preferably for not collaborating patients**

(30) Priority: 19.03.2003 IT rm20030123
(71) Applicant: Paragallo, Massimo, 00191 Roma (IT)
(72) Inventor: Paragallo, Massimo, 00191 Roma (IT)
(74) Representative: Iannone, Carlo Luigi

(57) **Abstract**

The invention concerns an improved radiogenic apparatus (1), particularly for orthopantomography of dental arches, preferably for not collaborating patients, comprising a base support (2), provided with wheels (3), a substantially vertical arm (4), provided, at a certain height, with a snood (6, allowing the orientation of the upper part of the same arm (4) substantially along a 180° arc, and an image acquisition head (5), provided at the end of said arm (4).

## Description

The present invention concerns an improved radiogenic apparatus, particularly for orthopantomography of dental arches, preferably for not collaborating patients.

More specifically, the invention concerns an apparatus of the above kind, suitably realised to allow to the operator to easily carry out the examination also on patients that are not able to adapt to the apparatus, as instead necessary with the present apparatuses and that really prevent each diagnostic and intra-surgery evaluation and diagnosis.

As it is well known, at present all the existing apparatuses destined to the orthopantomography examination of the dental arches require that the patient is in a vertical position.

Study on which the present invention is based starts from the analyses of this kind of apparatus, to transform the same in an apparatus able to operate according to a horizontal configuration.

Mainly, problems that have been faced when realising the apparatus according to the invention can be individuated in the fact that the apparatus according to the invention moves toward the patient and it is not the patient who has to move toward the apparatus, as it occurs traditionally, that a different geometry of the system must be realised involving very different mechanical loadings, and that must be provide a seat making vertical movements and with a head rest comprised with radio-transparent material.

It is therefore specific object of the present invention an improved radiogenic apparatus, particularly for orthopantomography of dental arches, preferably for not collaborating patients, comprising a base support, provided with wheels, a substantially vertical arm, provided, at a certain height, with a snood, allowing the orientation of the upper part of the same arm substantially along a 180° arc, and an image acquisition head, provided at the end of said arm.

Preferably, according to the invention, it will be provided a seat, on which the patient can be laid, with the parts interfering with the improved radiogenic apparatus comprised of radio-transparent material.

Furthermore, according to he invention, it is provided a slide to adjust the position of the acquisition head with respect to the patient, said slide allowing the adjustment according to a perpendicular and vertical direction with respect to the seat, and according to a longitudinal direction with respect to the patient.

Always according to the invention, it will be provided a disposable centring device in occlusion of the patient dental arches, preferably comprised of thermo-plastic paste and conformed to the dental surfaces.

Still according to the invention, said acquisition head will be provided with an interface for the horizontal rotation, particularly by suitable radial bearings, goniometric systems and wheels, and an actuator.

The present invention will be now described, for illustrative but not limitative purposes, according to its preferred embodiments, with particular reference to the figures of the enclosed drawings, wherein:
Figure 1 is a perspective view of an improved radiogenic apparatus according to the invention;
Figure 2 is a perspective view of the apparatus according to the invention in an operative mode, before its use; and
Figure 3 is a perspective view of the apparatus according to the invention in an operative mode, during its use.

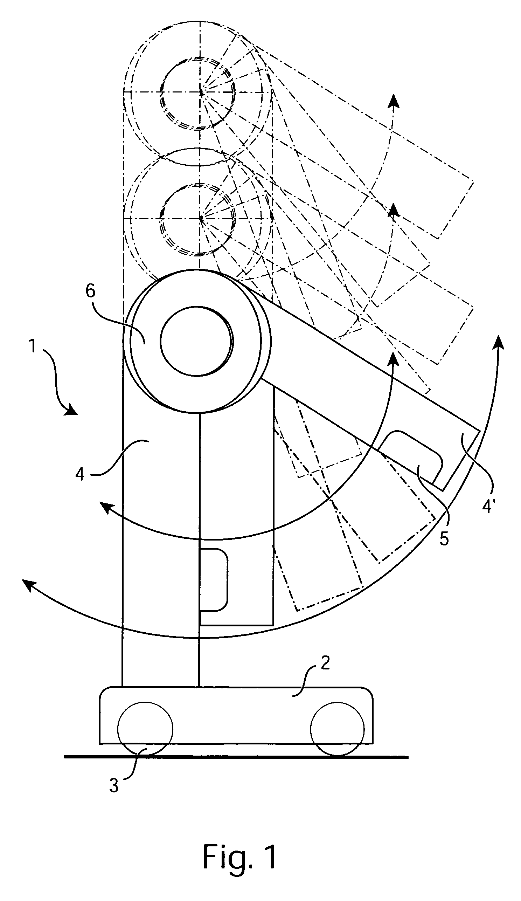

Observing now figures of the enclosed drawings, and particularly first figure 1, it is shown an apparatus 1 for orthopanoramic according to the invention, providing a movable support 2, with wheels 3, in such a way to allow its approaching to the patient already in a horizontal position and, eventually, under general anesthesia condition, maintained by a nose-tracheal intubation.

The vertical arm 4 is mounted on the movable support of the improved radiogenic apparatus, having on its upper end the orthopanoramic acquisition head 5.

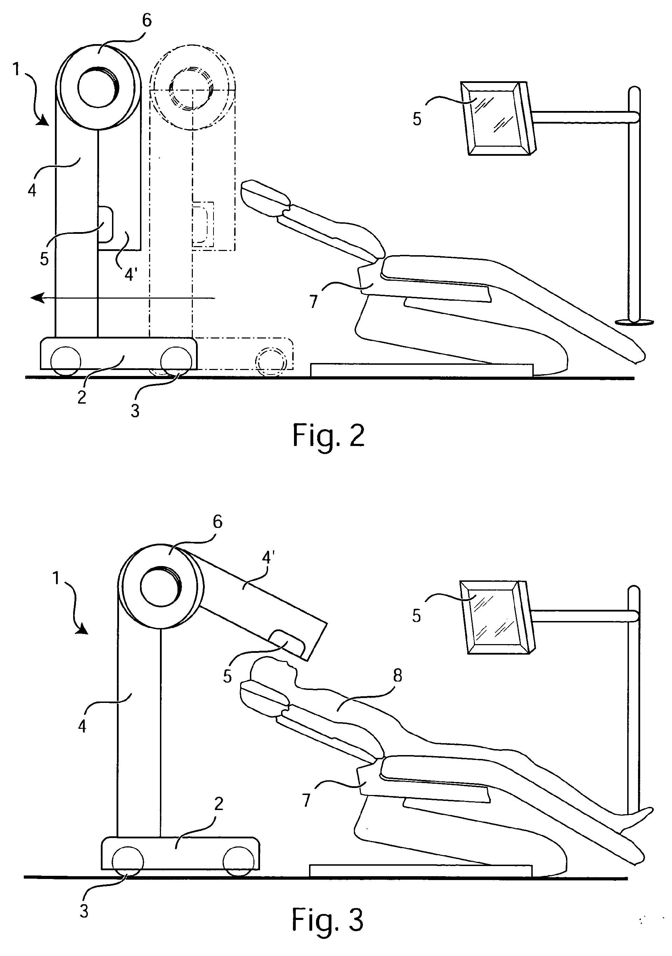

Centrally, said vertical arm 4 is provided with a snood 6, allowing to orient the upper part 4' of the arm 4, in such a way to make it taking the desired position with respect to the patient, as it will be more specifically described with reference to the figures 2 and 3, according to the arrows shown in figure 1.

Coming now to describe figures 2 and 3, it can be noted the positioning of the apparatus 1 according to the invention with respect to a seat 7 on which the patient 8 is seated. It is further shown a screen 9 for reading the information acquired by the head 5.

Patient 8 can be a self-sufficient patient, or a not collaborating patient, both to psychophysical or psychic disability of the same, or under general anesthesia condition.

Support on the wheel 3 will bring the apparatus in the desired horizontal position with an imprecision of about ± 20 mm, thanks to reference points for the wheels 3 drawn on the pavement of the room. Instead, vertical approaching will be carried out with a suitable movement of the seat 7.

Finally, so called fine adjustment, thus referred to the luminous sight lines of the apparatus head, will be realised thanks to a manually operated guide system, interposed between support 2 on wheels 3 and the source - plate assembly.

Said slide (not shown in detail) will allow the movement of the apparatus along the perpendicular direction and the vertical direction, by the movement of the seat 7, and along the longitudinal direction with respect to the same patient. Precision of this movement will correspond to the precision of standard radiographic apparatuses, and will depend by manual skill of the operator, and in any case the movement field will be such to completely cover the indeterminateness range defined by the movement on the wheels 3.

Finally, apparatus 1 according to the invention provides a disposable centring device in occlusion of the patient dental arches comprised of thermoplastic paste and conformed to the dental surfaces, exploiting for its stability the natural restraint of the dental undercut.

After the apparatus 1 has been centred on the proper focus of its elliptical trajectory, the source - plate system 5 will be rotated about the jaw of the patient 8. particularly, head 5 must be moved no more along a vertical axis, and thus in a condition in which the weight of the apparatus 1 beside being homogeneously distributed, does not overload the motor dedicated to the same movement, but along a horizontal axis, thus in a condition in which loading during the rotation is equal to the length of the axis to be moved for the real weight of the source - sensor assembly.

This kind of rotation will require the realisation of the interface for the horizontal rotation and thus the introduction of suitable radial bearings, goniometric systems and wheels, and also the replacement of the motor with a more powerful actuator.

The present invention has been described for illustrative but not limitative purposes, according to its preferred embodiments, but it is to be understood that modifications and/or changes can be introduced by those skilled in the art without departing from the relevant scope as defined in the enclosed claims.

## Claims

1. Improved radiogenic apparatus, particularly for orthopantomography of dental arches, preferably for not collaborating patients, **characterised in that** it comprises a base support, provided with wheels, a substantially vertical arm, provided, at a certain height, with a snood, allowing the orientation of the upper part of the same arm substantially along a 180° arc, and an image acquisition head, provided at the end of said arm.

2. Improved radiogenic apparatus, particularly for orthopantomography of dental arches, according to claim 1, **characterised in that** it is provided a seat, on which the patient can be laid.

3. Improved radiogenic apparatus, particularly for orthopantomography of dental arches, according to claim 2, **characterised in that** the parts of the seat interfering with the improved radiogenic apparatus are comprised of radio-transparent material.

4. Improved radiogenic apparatus, particularly for orthopantomography of dental arches, according to one of the preceding claims, **characterised in that** it is provided a slide to adjust the position of the acquisition head with respect to the patient.

5. Improved radiogenic apparatus, particularly for orthopantomography of dental arches, according to claim 4, **characterised in that** said slide allows the adjustment according to a perpendicular and vertical direction with respect to the seat, and according to a longitudinal direction with respect to the patient.

6. Improved radiogenic apparatus, particularly for orthopantomography of dental arches, according to one of the preceding claims, **characterised in that** it is provided a disposable centring device in occlusion of the patient dental arches.

7. Improved radiogenic apparatus, particularly for orthopantomography of dental arches according to claim 6, **characterised in that** disposable centring device in occlusione is comprised of thermo-plastic paste and conformed to the dental surfaces.

8. Improved radiogenic apparatus, particularly for orthopantomography of dental arches, according to one of the preceding claims, **characterised in that** said acquisition head is provided with an interface for the horizontal rotation.

9. Improved radiogenic apparatus, particularly for orthopantomography of dental arches according to claim 6, **characterised in that** said interface for the horizontal rotation is realised by suitable radial bearings, goniometric systems and wheels, and an actuator.

10. Improved radiogenic apparatus, particularly for orthopantomography of dental arches, according to each one of the preceding claims, substantially as illustrated and described.
